(19) Europäisches Patentamt
European Patent Office
Office européen des brevets

(11) **EP 4 654 246 A1**

(12) **EUROPEAN PATENT APPLICATION**

(43) Date of publication:
**26.11.2025 Bulletin 2025/48**

(51) International Patent Classification (IPC):
*H01J 49/00* (2006.01)　　　*G01N 33/68* (2006.01)
*G16B 40/10* (2019.01)　　　*G16C 20/20* (2019.01)

(21) Application number: **24178105.3**

(22) Date of filing: **24.05.2024**

(52) Cooperative Patent Classification (CPC):
**H01J 49/0036; G16B 40/10; G16C 20/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Bruker Switzerland AG
8117 Fällanden (CH)**

(72) Inventors:
- **PAPANASTASIOU, Dimitris**
  **8117 Fällanden (CH)**
- **ALEVISZOS, Georgios**
  **8117 Fällanden (CH)**
- **KOSMOPOULOU, Mariangela**
  **8117 Fällanden (CH)**
- **SUCKAU, Detlev**
  **8117 Fällanden (CH)**

(74) Representative: **Acapo AS
Edvard Griegs vei 1
5059 Bergen (NO)**

(54) **APPARATUS AND METHOD**

(57)　　A method of analyzing mass spectra, the method implemented by a computer including a processor and a memory, the method comprising steps:

(i) obtaining a mass spectrum comprising a series of isotope peak clusters, including a first isotope peak cluster and a second isotope peak cluster, corresponding to respective product ions of a precursor ion;

(ii) calculating theoretical isotope peak clusters for the series of isotope peak clusters;

(iii) fitting the theoretical isotope peak clusters to the series of isotope peak clusters;

(iv) ranking the theoretical isotope peak clusters, based on respective results of the fitting;

(v) selecting the theoretical isotope peak clusters, based on respective results of the ranking; and

(vi) generating a residual mass spectrum from the mass spectrum, using the selected theoretical isotope peak clusters;

(vii) repeating steps (iii) to (vi) for the residual mass spectrum; and

(viii) providing/outputting/storing the selected theoretical isotope peak clusters.

FIGURE 1

## Description

### FIELD

The technical field of the invention relates to methods of processing mass spectrometry data, for example mass spectra. For example, the invention may relate to methods of processing complex mass spectra generated by top-down mass spectrometry. For example, the invention may relate to methods of extracting sequence information from mass spectra of proteins and other classes of molecules *de novo.*

### BACKGROUND

Mass spectrometry (MS) is a powerful tool for analyzing proteins and other classes of biological macromolecules such as DNA/RNA and polysaccharides. Improvements in MS instrumentation offering enhanced sensitivity and mass resolving power have enabled the characterization of ions in the higher mass-to-charge (m/z) range. Top-down MS is concerned with the analysis of intact proteins subjected to gas phase fragmentation, yielding the molecular mass of the protein as well as protein ion fragments for deducing the primary structure of the protein. The level of information related to the primary structure of proteins has improved through the development of complementary dissociation methods, for example based on ion-electron and ion-photon interactions, leading to more complex and highly congested mass spectra containing a significantly increased number of fragments.

Despite the improvements in sample preparation, MS instrumentation and ion fragmentation methods, one of the most significant barriers for the proliferation of top-down MS workflows is the ability to analyze reliably the complex and highly congested mass spectra. In contrast to mainstream bottom-up MS where, for which a plethora of software tools has become available, software tools tailored to top-down MS data are lacking. The need for top-down MS software tools has become even more apparent in recent years since the efficiency of fragmentation methods, such as electron capture dissociation (ECD), electron induced dissociation (EID), ultra-violet photo-dissociation (UVPD), infrared multi-photon dissociation (IRMPD) and combinations thereof, has generated high quality but also remarkably complex top-down mass spectra. Hence, there is a clear need for improving the data analysis aspect of analytical workflows based on mass spectrometry.

Different approaches have been described to identify proteins based on tandem mass spectrometry (MS/MS) data. The most common approach is to utilize the available databases containing protein sequence information and/or to use existing libraries to match experimental MS/MS data. Spectral matching is typically applied for the identification of peptides that exhibit relatively simple fragment ion mass spectra where the monoisotopic mass of individual fragments can be accurately identified. In contrast, spectral matching becomes prone to errors as spectral complexity increases and monoisotopic peaks of higher m/z fragments are lost in the background. New methods are therefore necessary to measure the mass of the fragments reliably that consider the entire isotope clusters of the fragments and can further disentangle overlapping isotope distributions.

Moreover, the spectral matching approach suffers from several drawbacks and cannot be used for the identification of proteins from unknown genomes. Methods to directly extract the amino acid sequence information from the mass spectrum without the use of a database (*de novo* sequencing) have been developed. However, these are limited to peptides where spectra information is sparse. Sequencing heavily congested mass spectra *de novo* requires the development of new methods that account for the enhanced complexity due to extensive overlapping between isotope clusters. Furthermore, the excessive number of false positive de *novo* peptides from top down mass spectra must be reduced.

### SUMMARY OF THE INVENTION

A first aspect provides a computer-implemented method of analyzing mass spectrometry data, comprising steps:

(a) providing a mass spectrum containing fragment ion peaks and corresponding isotope clusters and determining centroid peaks therein;
(b) providing a set of theoretical isotope distributions in relation to the fragment ion peaks in the mass spectrum;
(c) adjusting the intensity of the provided theoretical isotope distributions to the intensity of the fragment ion peaks of the mass spectrum;
(d) providing a scoring system for ranking the theoretical isotope distributions based on the features of the mass spectrum;
(e) forming a subset of theoretical isotope distributions by selecting a single theoretical isotope distribution with the highest score for every individual fragment ion peak, optionally with each of the theoretical isotope distributions potentially assigned to more than one fragment ion peak;

(f) subtracting the intensities of the subset of the theoretical isotope distributions from the fragment ion peaks of the mass spectrum to produce a residual mass spectrum;

(g) providing the remaining set of theoretical isotope distributions from (b), while excluding the selected subset of distributions formed in (e); and

(h) repeating steps (c) to (g) for the residual mass spectrum mutatis mutandis until a set of convergence criteria is satisfied.

Additionally and/or alternatively, the first aspect provides a method of analyzing mass spectra, the method implemented by a computer including a processor and a memory, the method comprising:

(a) obtaining a mass spectrum including isotope peak clusters corresponding to respective product ions;

(b) relating theoretical isotope peak clusters corresponding to respective candidate product ions with the isotope peak clusters corresponding to the respective product ions;

(c) fitting respective intensities of the theoretical isotope peak clusters corresponding to the respective candidate product ions with the respective intensities of the isotope peak clusters corresponding to the respective product ions;

(d) ranking the theoretical isotope peak clusters corresponding to the respective candidate product ions for the isotope peak clusters corresponding to the respective product ions;

(e) identifying the respective product ions corresponding to the isotope peak clusters as the respective candidate product ions corresponding to the respective highest ranked theoretical isotope peak clusters;

(f) generating a residual mass spectrum including isotope peak clusters corresponding to respective product ions, using the respective highest ranked theoretical isotope peak clusters corresponding to the respective identified product ions;

(g) optionally, repeating steps (c) to (f) for the residual mass spectrum.

Additionally and/or alternatively, the first aspect provides a method of analyzing mass spectra, the method implemented by a computer including a processor and a memory, the method comprising steps:

(i) obtaining a mass spectrum comprising a series of isotope peak clusters, including a first isotope peak cluster and a second isotope peak cluster, corresponding to respective product ions of a precursor ion;

(ii) calculating theoretical isotope peak clusters for the series of isotope peak clusters;

(iii) fitting the theoretical isotope peak clusters to the series of isotope peak clusters;

(iv) ranking the theoretical isotope peak clusters, based on respective results of the fitting;

(v) selecting the theoretical isotope peak clusters, based on respective results of the ranking; and

(vi) generating a residual mass spectrum from the mass spectrum, using the selected theoretical isotope peak clusters;

(vii) optionally, repeating steps (iii) to (vi) for the residual mass spectrum; and

(viii) providing/outputting/storing the selected theoretical isotope peak clusters.

A second aspect provides a computer implemented method of determining chemical formulas for calculating the theoretical isotope distributions corresponding to the isotope clusters of the fragment ion peaks in the mass spectrum, wherein each chemical formula exhibits a theoretical mass approximating the mass of the corresponding isotope cluster, and further shifting the theoretical isotope distributions accordingly to align on the mass or m/z axis with the isotope clusters.

In one example, the method provides theoretical isotope distributions having the same charge state compared to the corresponding isotope clusters of the fragment ion peaks.

A third aspect provides a computer implemented *de novo* sequencing method for analyzing a mass spectrum, comprising:

wherein the mass spectrum containing isotope clusters and the corresponding monoisotopic fragment ion peaks is produced from a precursor ion originating from an unknown molecular identity, for example a protein with an unknown amino acid sequence.

A fourth aspect provides a computer implemented sequence confirmation method for analyzing a mass spectrum comprising:

wherein the fragment ion mass spectrum is produced from a precursor ion originating from a partially known molecular identity, for example a protein with a partially known amino acid sequence, or a known protein with unknown or partially known post translational modifications.

A fifth aspect provides a computer implemented method of identifying a molecule by processing the corresponding mass spectrum, according to any one or more of the first to fourth aspects.

A sixth aspect of the present invention provides a computer implemented method of identifying a molecule by annotating the fragment ion peaks in the corresponding mass spectrum, according to any one or more of the first to fifth aspects.

A seventh aspect provides a computer implemented method of identifying, at least partially, the amino acid sequence of a protein, according to any one or more of the first to sixth aspects.

An eighth aspect provides a computer implemented method of structurally characterizing a molecule, according to any one or more of the first to sevemth aspects.

A ninth aspect provides a computer implemented method for identifying a molecule by analyzing the corresponding fragment ion mass spectrum, comprising:

wherein the fragment ion mass spectrum is produced from an unknown molecule having a mass listed in a database containing at least a series of different molecules having comparable ion masses within a predetermined mass tolerance, and further analyzing the fragment ion mass spectrum according to the first, and/or second, and/or fourth, and/or sixth aspects applied to all the different molecules in the series to find the best match and identify the molecule from the list in the database.

A tenth aspect provides a computer implemented method for identifying a molecule by analyzing the corresponding fragment ion mass spectrum, comprising:

wherein the fragment ion mass spectrum is produced from an unknown molecule having a mass and a corresponding fragment ion mass spectrum listed in a spectral library containing at least a series of different molecules having comparable ion masses within a predetermined mass tolerance and corresponding fragment ion mass spectra, and further analyzing the fragment ion mass spectrum from the unknown analyte according to the first, and/or second, and/or fourth, and/or sixth aspects applied to all the different molecules in the series and identify the molecule by finding the best fragmentation mass spectrum in the library that matches the fragment ion mass spectrum.

An eleventh aspect provides a computer implemented method including a graph for analyzing a mass spectrum *de novo,* the graph comprising a set of nodes connected through a set of edges, with each node representing the monoisotopic mass of a (theoretical) isotope distribution, and with the edges of the graph representing the difference in the (monoisotopic) mass between two nodes.

A twelfth aspect provides a computer implemented method for reducing the number of false positive *de novo* peptides listed according to the eleventh aspect, the method further comprising any one, two or three of:

(a) filtering the list by rejecting *de novo* peptides that do not satisfy a first condition, the first condition comprising: calculating the total mass of a first pair of nodes corresponding to a known precursor mass, the first pair including a first node and a second node, calculating a second pair of nodes corresponding to the same precursor mass, the second pair including another first node and another second node, with the mass difference between the first nodes and the second nodes corresponding to the same mass in the list of masses, for example, to one of the masses of the standard twenty amino acids;

(b) filtering the list by rejecting *de novo* peptides that do not satisfy a second condition, the second condition comprising: calculating a first mass difference between a first node in a first pair of nodes and a second node in a second pair of nodes, calculating a second mass difference between the second node in the first pair of nodes and the second node in the second pairs of nodes, with the first and second mass differences corresponding to the mass difference between two primary fragments, for example, the mass difference between a "b" fragment type and an "a" fragment type;

(c) filtering the list by rejecting *de novo* peptides that do not satisfy a third condition, the third condition comprising: calculating a first pair of nodes corresponding to a mass in the list of masses, calculating a second pair of nodes corresponding to the same mass in the list of masses, with a first node of the first pair of nodes and a first node of the second pair of nodes having the same (neutral) mass, and with the second node of the first pair of nodes and the second node of the second pair of nodes also having the same (neutral) mass.

A thirteenth aspect provides a computer implemented method for reducing the number of false positive *de novo* peptides listed by combining any two of the three filtering aspects of the twelfth aspect and optionally, combining all three filtering aspects of the twelfth aspect to produce a list of *de novo* peptides.

A fourteenth aspect provides a method of interpreting mass spectra, the method implemented by a computer including a processor and a memory, the method comprising steps:

(1) obtaining theoretical isotope peak clusters for a mass spectrum, for example by analyzing the mass spectrum according to the first aspect;

(2) mutually associating the theoretical isotope peak clusters, based on a set of predetermined masses, for example the masses of the twenty amino acids and/or modifications thereof;

(3) mutually cross-associating the mutually associated theoretical isotope peak clusters, based on precursor ion information and/or product ion information, for example product ion patterns; and

(4) interpreting the mass spectrum, based on the mutually cross-associated theoretical isotope peak clusters.

A fifteenth aspect provides a method implemented by a computer including a processor and a memory, the method comprising:

analyzing a mass spectrum according to any one or more of the first aspect to the tenth aspect;
interpreting the mass spectrum according to any one or more of the eleventh aspect to the fourteenth aspect;
proposing a candidate molecule or molecular species, for example a protein, by database searching the results of the interpreting, such as a peptide, resulting from the mutually cross-associated theoretical isotope clusters; and
optionally, confirming the candidate molecule or molecular species, by matching, for example fragment matching, using the mass spectrum.

A sixteenth aspect provides: a computer including a processor and a memory configured to perform a method according to any one or more of the first aspect to the fifteenth aspect; a computer program comprising instructions which, when executed by a computer including a processor and a memory, cause the computer to perform a method according to any one or more of the first aspect to the fifteenth aspect; and/or a non-transient computer-readable storage medium comprising instructions which, when executed by a computer including a processor and a memory, cause the computer to perform a method according to any one or more of the first aspect to the fifteenth aspect.

A seventeenth aspect provides a mass spectrometer comprising a computer including a processor and a memory configured to perform a method according to any one or more of the first aspect to the fifteenth aspect.

## DETAILED DESCRIPTION OF THE INVENTION

The first aspect provides a computer-implemented method of analyzing mass spectrometry data, comprising steps:

(a) providing a mass spectrum containing fragment ion peaks and corresponding isotope clusters and determining centroid peaks therein;
(b) providing a set of theoretical isotope distributions in relation to the fragment ion peaks in the mass spectrum;
(c) adjusting the intensity of the provided theoretical isotope distributions to the intensity of the fragment ion peaks of the mass spectrum;
(d) providing a scoring system for ranking the theoretical isotope distributions based on the features of the mass spectrum;
(e) forming a subset of theoretical isotope distributions by selecting a single theoretical isotope distribution with the highest score for every individual fragment ion peak, optionally with each of the theoretical isotope distributions potentially assigned to more than one fragment ion peak;
(f) subtracting the intensities of the subset of the theoretical isotope distributions from the fragment ion peaks of the mass spectrum to produce a residual mass spectrum;
(g) providing the remaining set of theoretical isotope distributions from (b), while excluding the selected subset of distributions formed in (e); and
(h) repeating steps (c) to (g) for the residual mass spectrum mutatis mutandis until a set of convergence criteria is satisfied.

The analysis according to the first aspect provides a list or a compilation of theoretical isotope distributions that best explain the fragment ions peaks and corresponding isotope clusters in the mass spectrum. The theoretical isotope distributions disentangle the congested regions of overlapping isotope clusters with high precision, also providing an accurate measurement of the monoisotopic mass of all the isotope clusters corresponding to fragments.

*Relation - step (b)*

In one example, the method comprises determining the respective monoisotopic masses of the isotope peak clusters corresponding to the fragments.

In one example, the relation of the theoretical isotope distributions is based on the mass, for example the monoisotopic mass, and the charge state of the fragment ion peaks.

In one example, the theoretical isotope distributions are calculated based on chemical formulas having a mass approximating the mass, for example the monoisotopic mass, of the fragment ion peaks.

In one example, the theoretical isotope distributions are calculated based on the charge state of the fragment ion peaks.

In one example, the theoretical isotope distributions are shifted in the m/z scale to align with the fragment ion peaks.

In one example, the set of theoretical isotope distributions related to the isotope clusters in the mass spectrum fall within a predefined mass or m/z tolerance.

In one example, the method comprises adjusting the intensity of the theoretical distributions within a predetermined range relative to the intensity of the corresponding fragment ion peaks.

*Scoring system - step (d)*

In one example, the scoring system for ranking the theoretical isotope distributions is based on the mass difference and/or the mass-to-charge ratio difference between a theoretical isotope peak and a corresponding fragment ion peak.

In one example, the scoring system for ranking the theoretical isotope distributions is based on differences in the relative abundance between at least two neighboring theoretical isotope peaks and the corresponding fragment ion peaks.

In one example, the scoring system for ranking the theoretical isotope distributions is based on differences in the intensity and/or in the area-under-the-curve between the isotope clusters containing the fragment ion peaks and the corresponding theoretical isotope distributions.

In one example, the scoring system for ranking the theoretical isotope distributions is based on the correlation in mass accuracy and/or in ion intensity between the isotope clusters containing the fragment ion peaks and the corresponding theoretical isotope distributions.

*Convergence criteria - step (h)*

In one example, the residual mass spectrum contains no remaining fragment ion peaks above a predetermined threshold, for example a predetermined intensity and/or signal-to-noise (s/n) threshold, for the set of convergence criteria to be satisfied.

In one example, the relative change in the difference in the intensities of the theoretical isotope distributions and the intensities of the fragment ion peaks in two consecutive iterations falls below a predetermined threshold, for example a predetermined intensity and/or signal-to-noise (s/n) threshold, for the set of convergence criteria to be satisfied.

*Mass spectrum - step (a)*

In one example, the mass spectrum containing fragment ion peaks is acquired from a precursor ion originating from an unknown and/or an unidentified molecule or molecular species, for example a protein with an unknown and/or an unidentified amino acid sequence.

In one example, the mass spectrum containing fragment ion peaks is acquired from a precursor ion originating from a partially known and/or a partially identified molecule or molecular species, for example: a protein with a partially known and/or a partially identified amino acid sequence; a known protein with unknown and/or unidentified post translational modifications.

In one example, the method provides and/ or is a method of identification and/or structural characterization of a molecule or molecular species by analyzing the mass spectrum containing fragment ion peaks.

In one example, the method provides and/ or is a method of partial identification of the amino acid sequence of a protein.

In one example, the method provides and/ or is a method of identification of an unknown and/or unidentified molecule or molecular species by searching a list of theoretical fragment ion mass spectra to match the mass spectrum containing fragment ion peaks, wherein the list of theoretical mass spectra is calculated from a database comprising known and/or identified molecules or molecular species having comparable masses to the mass of the unknown and/or unidentified molecule or molecular species.

In one example, the method provides and/ or is a method of identification of an unknown and/or unidentified molecule or molecular species by searching a library of theoretical fragment ion mass spectra to match the mass spectrum containing fragment ion peaks.

Additionally and/or alternatively, the first aspect provides a method of analyzing mass spectra, the method implemented by a computer including a processor and a memory, the method comprising:

(a) obtaining a mass spectrum including isotope peak clusters corresponding to respective product ions;
(b) relating candidate theoretical isotope peak clusters with the isotope peak clusters;
(c) fitting respective intensities of the candidate theoretical isotope peak clusters to the respective intensities of the isotope peak clusters;

(d) ranking the candidate theoretical isotope peak clusters, based on a result of the fitting, for example using a scoring system;

(e) generating a residual mass spectrum from the mass spectrum, using the highest ranked candidate theoretical isotope peak clusters; and

(f) optionally, repeating steps (b) to (e) for the residual mass spectrum.

Additionally and/or alternatively, the first aspect provides a method of analyzing mass spectra, the method implemented by a computer including a processor and a memory, the method comprising steps:

(i) obtaining a mass spectrum comprising a series of isotope peak clusters, including a first isotope peak cluster and a second isotope peak cluster, corresponding to respective product ions of a precursor ion;

(ii) calculating theoretical isotope peak clusters for the series of isotope peak clusters;

(iii) fitting the theoretical isotope peak clusters to the series of isotope peak clusters;

(iv) ranking the theoretical isotope peak clusters, based on respective results of the fitting;

(v) selecting the theoretical isotope peak clusters, based on respective results of the ranking; and

(vi) generating a residual mass spectrum from the mass spectrum, using the selected theoretical isotope peak clusters;

(vii) optionally, repeating steps (iii) to (vi) for the residual mass spectrum; and

(viii) providing/outputting/storing the selected theoretical isotope peak clusters.

In one example, the first isotope peak cluster and the second isotope peak cluster mutually overlap, for example partially and/or wholly. That is, respective mass spectral peaks of the first isotope peak cluster and the second isotope peak cluster may have the same or similar m/z, though different intensities, for example. For example, isotope peak clusters for different charge states of a product ion may overlap, such as isotope peak clusters of a 2+ product ion may wholly overlap with isotope peak clusters for a corresponding 4+ product ion. For example, isotope peak clusters for different product ions may overlap.

In one example, the series of isotope peak clusters includes n isotope peak clusters, wherein n is a natural number greater than or equal to 2, for example 2, 3, 4, 5, 6, 7, 8, 9, 10, 20, 30, 40, 50, 100, 200, 300, 400, 500, 1000, 2000, 5000, 10000, 20000, 50000, 100000 or more. That is, the mass spectrum may be complex, including many, for example overlapping, isotope peak clusters.

In one example, the precursor ion comprises and/or is a single precursor ion, for example having a single neutral mass. For example, the precursor ion may be selected during mass spectrometry for top down analysis.

In one example, the series of isotope peak clusters includes product ions having the same mass and different charge states.

In one example, the mass spectrum comprises a second series of isotope peak clusters, including a first isotope peak cluster and a second isotope peak cluster, corresponding to respective product ions of a second precursor ion.

In one example, the mass spectrum comprises N series of isotope peak clusters, respectively including a first isotope peak cluster and a second isotope peak cluster, corresponding to respective product ions of respective precursor ions, wherein N is a natural number greater than or equal to 1, for example 1, 2, 3, 4, 5, 6, 7, 8, 9, 10, 20, 30, 40, 50, 100 or more

In one example, the method comprises centroiding the mass spectrum and/or decharging the mass spectrum, for example before step (ii). In this way, the theoretical isotope peak clusters are calculated for the centroided and/or decharged mass spectrum.

In one example, obtaining the mass spectrum comprises acquiring the mass spectrum.

In one example, calculating the theoretical isotope peak clusters for the series of isotope peak clusters comprises calculating the theoretical isotope peak clusters for a plurality of peaks, optionally for each peak, of the series of isotope peak clusters.

In one example, calculating the theoretical isotope peak clusters for the series of isotope peak clusters comprises calculating the theoretical isotope peak clusters for the respective masses and/or charge states of the series of isotope peak clusters, for example for a plurality of peaks, optionally for each peak, of the series of isotope peak clusters.

In one example, calculating the theoretical isotope peak clusters for the series of isotope peak clusters comprises predicting respective chemical formulae for the series of isotope peak clusters, for example for a plurality of peaks, optionally for each peak, of the series of isotope peak clusters.

In one example, fitting the theoretical isotope peak clusters to the series of isotope peak clusters comprises shifting, optionally aligning, the theoretical isotope peak clusters relative to the series of isotope peak clusters (i.e. m/z).

In one example, fitting the theoretical isotope peak clusters to the series of isotope peak clusters comprises scaling (i.e. intensities) the theoretical isotope peak clusters relative to the series of isotope peak clusters.

In one example, fitting the theoretical isotope peak clusters to the series of isotope peak clusters comprises iteratively and/or simultaneously fitting the theoretical isotope peak clusters to the series of isotope peak clusters.

In one example, generating the residual mass spectrum from the mass spectrum, using the theoretical isotope peak clusters, comprises determining a residual value for the residual mass spectrum.

In one example, repeating steps (iii) to (vi) for the residual mass spectrum comprises repeating steps (iii) to (vi) for the residual mass spectrum while the residual value is greater than a predetermined threshold.

For example, the method according to the first aspect may be as described below:

Start with a peak picked spectrum **S** and a set of theoretical distributions **D** (can be derived from the Averagine chemical formula or directly via computational fragmentation of a protein). A copy of the spectrum **S** is denoted as **S'.**

The goal of the algorithm is to obtain an optimal set Ds that ideally constitutes the minimum number of selected distributions from **D** that best explain the spectrum **S.**

(a) Initially, the intensities of all theoretical distributions are adjusted to the spectrum **S** individually, that is, overlapping between them is not considered. The intensity adjustment can be performed using any curve fitting algorithm (Levenberg-Marquardt etc.)

(b) Score all the distributions from **D** using our scoring system and store their scores in a vector **Q.** Assume that the higher the score the better.

Until convergence, do:

1) For each centroid in the spectrum **S',** find out which theoretical distribution centroids from **D** are aligned to it according to a specified mass accuracy parameter.

2) For each centroid in **S',** determine the theoretical distribution that has a theoretical centroid aligned to it and has the maximum score, taken from **Q.** Gather all these aligned theoretical centroids into a set **Vc** and for each theoretical distribution find the theoretical centroids that are present in **Vc** and calculate the ratio between the sum of intensities of their theoretical centroids in **Vc** and the sum of intensities of all the centroids of the theoretical distribution. According to the value of this ratio, either choose to reject or place each theoretical distribution in the set of selected distributions **Ds,** removing it from **D.**

3) Adjust the intensities of all the theoretical distributions in **Ds** such that their sum adds up as close as possible to the intensity of the original spectrum **S.** This computational problem is of the form Ax = b where A are the intensities of the theoretical distributions, x is the factor to be multiplied to each distribution and b is the intensity vector of the spectrum **S.** All values of x need to be non-negative. The algorithm used to solve this problem is called **NNLS** (Non-negative least squares).

4) Sum all intensities of the distributions in **Ds** and gather them in vector **I,** which has the same length as the intensity vector of the spectrum **S'.** Subtract I from **S'** to end up with a residual spectrum.

5) Perform peak picking on the spectrum **S'** ending up with a new set of centroids.

6) Individually adjust the intensities of all remaining theoretical distributions in the set **D** (meaning that overlapping is not considered) and score them, replacing their scores in the set **Q.**

7) The process is terminated either if there are no more remaining distributions **(D** is empty) or if the relative change between the intensity coverage of the spectrum **S** across iterations is below a user defined threshold. (defaults at 1e-3)

The second aspect provides a computer implemented method of determining chemical formulas for calculating the theoretical isotope distributions corresponding to the isotope clusters of the fragment ion peaks in the mass spectrum, wherein each chemical formula exhibits a theoretical mass approximating the mass of the corresponding isotope cluster, and further shifting the theoretical isotope distributions accordingly to align on the mass or m/z axis with the isotope clusters.

In one example, the method provides theoretical isotope distributions having the same charge state compared to the corresponding isotope clusters of the fragment ion peaks.

In one example, the method provides theoretical isotope distributions calculated based on the Averagine chemical formula for associating and aligning with the isotope clusters of the peptide or protein fragment ion peaks.

The third aspect provides a computer implemented *de novo* sequencing method for analyzing a mass spectrum, comprising:

wherein the mass spectrum containing isotope clusters and the corresponding monoisotopic fragment ion peaks is produced from a precursor ion originating from an unknown molecular identity, for example a protein with an unknown amino acid sequence.

The fourth aspect provides a computer implemented sequence confirmation method for analyzing a mass spectrum comprising:

wherein the fragment ion mass spectrum is produced from a precursor ion originating from a partially known molecular identity, for example a protein with a partially known amino acid sequence, or a known protein with unknown or partially known post translational modifications.

The fifth aspect provides a computer implemented method of identifying a molecule by processing the corresponding mass spectrum, according to the first, second and/or third aspects.

The sixth aspect of the present invention provides a computer implemented method of identifying a molecule by annotating the fragment ion peaks in the corresponding mass spectrum according to the first, second, third and/or fourth aspects.

In one example, the method comprises providing a chemical formula to calculate theoretical isotope distributions of the fragments based on a lead sequence related to the partially known molecular identity.

In one example, the method comprises modifying the lead sequence to further analyze the mass spectrum and identify/characterize the molecule and its structure.

The seventh aspect provides a computer implemented method of identifying, at least partially, the amino acid sequence of a protein, according to any one or more aspects.

The eighth aspect provides a computer implemented method of structurally characterizing a molecule, according to any one or more aspects.

The ninth aspect provides a computer implemented method for identifying a molecule by analyzing the corresponding fragment ion mass spectrum, comprising:

wherein the fragment ion mass spectrum is produced from an unknown molecule having a mass listed in a database containing at least a series of different molecules having comparable ion masses within a predetermined mass tolerance, and further analyzing the fragment ion mass spectrum according to the first, and/or second, and/or fourth, and/or sixth aspects applied to all the different molecules in the series to find the best match and identify the molecule from the list in the database.

The tenth aspect provides a computer implemented method for identifying a molecule by analyzing the corresponding fragment ion mass spectrum, comprising:

wherein the fragment ion mass spectrum is produced from an unknown molecule having a mass and a corresponding fragment ion mass spectrum listed in a spectral library containing at least a series of different molecules having comparable ion masses within a predetermined mass tolerance and corresponding fragment ion mass spectra, and further analyzing the fragment ion mass spectrum from the unknown analyte according to the first, and/or second, and/or fourth, and/or sixth aspects applied to all the different molecules in the series and identify the molecule by finding the best fragmentation mass spectrum in the library that matches the fragment ion mass spectrum.

The eleventh aspect provides a computer implemented method including a graph for analyzing a mass spectrum *de novo,* the graph comprising a set of nodes connected through a set of edges, with each node representing the monoisotopic mass of a (theoretical) isotope distribution, and with the edges of the graph representing the difference in the (monoisotopic) mass between two nodes.

For example, generating a list of *de novo* peptides from a fragment ion mass spectrum may comprise steps of:

(a) processing or analyzing a mass spectrum, for example based on the first aspect and/or the second aspect, to identify, for example accurately identify e.g. within 10 ppm or within 5 ppm or within 1 ppm for example within 0.5 ppm mass tolerance, the monoisotopic masses of the fragments;

(b) providing a list of masses corresponding, for example, to the masses of the standard twenty amino acids and/or modifications thereof;

(c) calculating the mass difference between any two nodes and generating a graph with the set of edges matching the list of masses;

(d) starting from a root node representing a node with the smallest monoisotopic mass within one or more node paths originating from the root node, and with the edges connecting the nodes defining the one or more node paths corresponding to the list of masses; and

(e) providing a list of *de novo* peptides, with every peptide corresponding to a single node path within the graph.

In one example, the eleventh aspect provides a computer implemented method of *de novo* analyzing a protein mass spectrum, comprising:

(a) providing a mass spectrum containing fragment ion peaks and corresponding isotope clusters and determining centroid peaks therein;

(b) matching a set of theoretical isotope distributions to the fragment ion peaks in the mass spectrum;

(c) providing a list of masses corresponding, for example, to the masses of the standard twenty amino acids;

(d) generating a graph comprising a set of nodes, with each node representing the monoisotopic mass of a theoretical isotope distribution, and with the set of nodes connected through edges representing the difference in the monoisotopic mass between two nodes; wherein the edges match the list of masses, for example, the masses of the standard twenty amino acids;

(e) identifying a root node representing a node with the smallest monoisotopic mass within a one or more node paths originating from the root node, and with the edges connecting the nodes defining the one or more node paths corresponding to the list of masses, for example, to the masses of the standard twenty amino acids; and

(f) providing a list of *de novo* peptides, with every peptide corresponding to a single node path within the graph.

In one example, the list of *de novo* peptides is reduced to the peptides satisfying a first condition, the first condition comprising:

calculating the total mass of a first pair of nodes corresponding to a known precursor mass, the first pair including a first node and a second node, calculating a second pair of nodes corresponding to the same precursor mass, the second pair including another first node and another second node, with the mass difference between the first nodes and the second nodes corresponding to the same mass in the list of masses, for example, to one of the masses of the standard twenty amino acids;

In one example, the list of *de novo* peptides is reduced to the peptides satisfying a second condition, the second condition comprising:

calculating a first mass difference between a first node in a first pair of nodes and a second node in a second pair of nodes, calculating a second mass difference between the second node in the first pair of nodes and the second node in the second pairs of nodes, with the first and second mass differences corresponding to the mass difference between two primary fragments, for example, the mass difference between a "b" fragment type and an "a" fragment type.

In one example, the list of *de novo* peptides is reduced to the peptides satisfying a third condition, the third condition comprising:

calculating a first pair of nodes corresponding to a mass in the list of masses, calculating a second pair of nodes corresponding to the same mass in the list of masses, with a first node of the first pair of nodes and a first node of the second pair of nodes having the same (neutral) mass, and with the second node of the first pair of nodes and the second node of the second pair of nodes also having the same (neutral) mass.

In one example, the list of *de novo* peptides is reduced by applying any two of the three conditions.

In one example, the list of *de novo* peptides is reduced by applying all three conditions.

The twelfth aspect provides a computer implemented method for reducing the number of false positive *de novo* peptides listed according to the eleventh aspect, the method further comprising any one, two or three of:

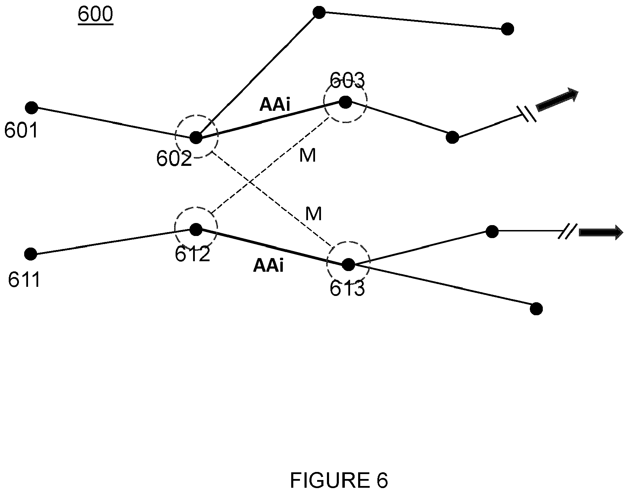

(a) filtering the list by rejecting *de novo* peptides that do not satisfy a first condition (described herein with respect to Figure 6 as the complementarity or bidirectional rule), the first condition comprising:

calculating the total mass of a first pair of nodes corresponding to a known precursor mass, the first pair including a first node and a second node, calculating a second pair of nodes corresponding to the same precursor mass, the second pair including another first node and another second node, with the mass difference between the first nodes and the second nodes corresponding to the same mass in the list of masses, for example, to one of the masses of the standard twenty amino acids;

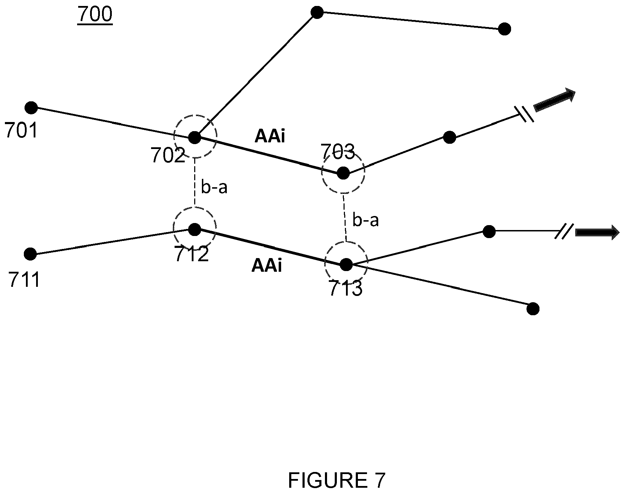

(b) filtering the list by rejecting *de novo* peptides that do not satisfy a second condition (described herein with respect to Figure 7 as the unidirectional or fragment delta rule), the second condition comprising:

calculating a first mass difference between a first node in a first pair of nodes and a second node in a second pair of nodes, calculating a second mass difference between the second node in the first pair of nodes and the second node in the second pairs of nodes, with the first and second mass differences corresponding to the mass difference between two primary fragments, for example, the mass difference between a "b" fragment type and an "a" fragment type;

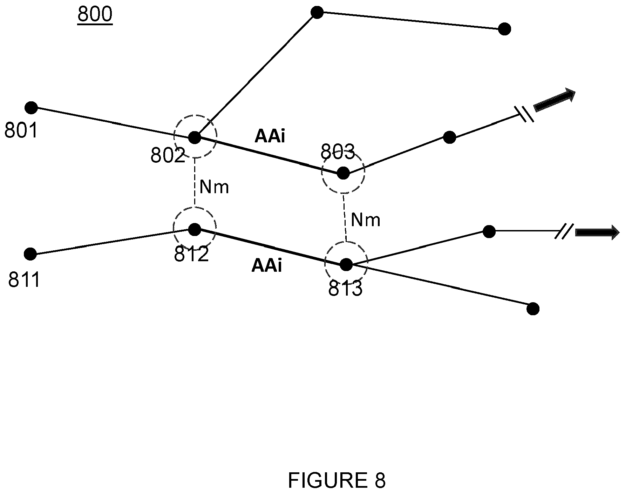

(c) filtering the list by rejecting *de novo* peptides that do not satisfy a third condition (described herein with respect to Figure 8 as the multi-z or charge-state rule), the third condition comprising:

calculating a first pair of nodes corresponding to a mass in the list of masses, calculating a second pair of nodes corresponding to the same mass in the list of masses, with a first node of the first pair of nodes and a first node of the second pair of nodes having the same (neutral) mass, and with the second node of the first pair of nodes and the second node of the second pair of nodes also having the same (neutral) mass;

For the third condition to be satisfied, the first nodes in both pair of nodes correspond to different m/z ratios with the same neutral mass at different charge states, and the second nodes in both pair of nodes correspond to different m/z ratios with another same neutral mass at different charge states.

The thirteenth aspect provides a computer implemented method for reducing the number of false positive *de novo* peptides listed by combining any two of the three filtering aspects of the twelfth aspect and optionally, combining all three filtering aspects of the twelfth aspect to produce a list of *de novo* peptides.

The fourteenth aspect provides a method of interpreting mass spectra, the method implemented by a computer including a processor and a memory, the method comprising steps:

(1) obtaining theoretical isotope peak clusters for a mass spectrum, for example by analyzing the mass spectrum according to the first aspect;
(2) mutually associating the theoretical isotope peak clusters, based on a set of predetermined masses, for example the masses of the twenty amino acids and/or modifications thereof;
(3) mutually cross-associating the mutually associated theoretical isotope peak clusters, based on precursor ion information and/or product ion information, for example product ion patterns; and
(4) interpreting the mass spectrum, based on the mutually cross-associated theoretical isotope peak clusters.

That is, the theoretical isotope peak clusters are mutually associated (for example, related or linked) by differences in respective masses (i.e. the set of predetermined masses). That is, the theoretical isotope peak clusters are mutually associated in a first dimension, for example to provide unbranched or branched node paths, having respective root nodes. For example, a candidate peptide may correspond to mutually associated theoretical isotope peak clusters (i.e. mutually associated in a first dimension, for example to provide unbranched or branched node paths, such as a single node path).

That is, the mutually associated theoretical isotope peak clusters are mutually cross-associated (for example, cross-related or cross-linked) by respective masses (e.g. precursor ion information and/or product ion information, for example product ion patterns). That is, the mutually associated theoretical isotope peak clusters (i.e. mutually associated in a first dimension, for example to provide unbranched or branched node paths) are mutually cross-associated in a second dimension, for example to provide cross-referenced unbranched or branched node paths. For example, a higher scored or confirmed peptide may correspond to mutually cross-associated theoretical isotope peak clusters (i.e. mutually associated in a first dimension, for example to provide unbranched node paths, such as a single unbranched node path, and mutually cross-associated in a second dimension). In other words, candidate peptides may be higher scored or confirmed by this mutual cross-association, for example.

In this way, the mass spectrum is interpreted based on the mutually cross-associated theoretical isotope peak clusters, for example to identify, at least in part, the molecule or molecular species. For example, by higher scoring or confirming peptides in this way, the number of false positives is significantly reduced since the mass spectrum may be interpreted using only cross-associated (i.e. confirmed) candidate peptides, thereby improving confidence in identifying, at least in part, the sequence of the protein.

The method according to the fourteenth aspect may include any of the steps of the eleventh aspect, the twelfth aspect and/or the thirteenth aspect, mutatis mutandis.

In one example, the method comprises performing steps (2) and (3) successively or consecutively, for example in order and/or in reverse order.

In one example, the method comprises performing steps (2) and (3) simultaneously or concurrently.

In one example, the method comprises performing steps (2) and (3) iteratively.

In one example, mutually cross-associating the mutually associated theoretical isotope peak clusters, based on precursor ion information and/or product ion information, for example product ion patterns comprises mutually cross-associating only the mutually associated theoretical isotope peak clusters, based on precursor ion information and/or product ion information, for example product ion patterns. For example, theoretical isotope peak clusters that are not mutually associated may be excluded, for example during step (2), after step (2), before step (3) and/or during step (3). For example, only theoretical isotope peak clusters that are mutually associated may be included (i.e. retained), for example during step (2), after step (2), before step (3) and/or during step (3).

In one example, interpreting the mass spectrum, based on the mutually cross-associated theoretical isotope peak clusters, comprises interpreting the mass spectrum, based on the only mutually cross-associated theoretical isotope peak clusters. For example, mutually associated theoretical isotope peak clusters that are not mutually cross-associated may be excluded, for example during step (3), after step (3), before step (4) and/or during step (4). For example, only mutually associated theoretical isotope peak clusters that are mutually cross-associated may be included (i.e. retained), for example during step (3), after step (3), before step (4) and/or during step (4).

In one example, the method comprises filtering the mutually associated theoretical isotope peak clusters to retain only the mutually cross-associated theoretical isotope peak clusters, for example during step (3), after step (3), before step (4) and/or during step (4).

In one example, mutually associating the selected theoretical isotope peak clusters, based on the set of predetermined masses, comprises mutually associating the selected theoretical isotope peak clusters, based on the set of predetermined masses comprising the masses of the twenty amino acids.

In one example, mutually associating the selected theoretical isotope peak clusters, based on the set of predetermined masses, comprises mutually associating the selected theoretical isotope peak clusters, wherein a difference between respective monoisotopic masses of two mutually associated selected theoretical isotope peak clusters is a predetermined mass of the set of predetermined masses.

In one example, mutually associating the selected theoretical isotope peak clusters, based on the set of predetermined masses, comprises generating a graph comprising a set of nodes, with each node representing the monoisotopic mass of a theoretical isotope distribution, and with the set of nodes connected through edges representing the difference in the monoisotopic mass between two nodes; wherein the edges match the list of masses, for example, the masses of the standard twenty amino acids.

In one example, interpreting the mass spectrum, based on the mutually cross-associated theoretical isotope peak clusters, comprises determining sequence information, based on the mutually cross-associated theoretical isotope peak clusters.

In one example, the method comprises filtering the mutually associated theoretical isotope peak clusters to retain only the mutually associated theoretical isotope peak clusters meeting the first condition, the second condition and/or the third condition.

In one example, the method comprises filtering the mutually associated theoretical isotope peak clusters to retain only the mutually associated theoretical isotope peak clusters wherein:

the total mass of a first pair of mutually associated theoretical isotope peak clusters corresponds to a known precursor mass;

the total mass of a second pair of mutually associated theoretical isotope peak clusters corresponds to the known precursor mass; and

a mass difference between the respective mutually associated theoretical isotope peak clusters of the first pair of mutually associated theoretical isotope peak clusters and the second pair of

mutually associated theoretical isotope peak clusters corresponds to the same mass in the set of predetermined masses.

This corresponds with the first condition.

In one example, the method comprises filtering the mutually associated theoretical isotope peak clusters to retain only the mutually associated theoretical isotope peak clusters wherein:

a first mass difference between non-respective mutually associated theoretical isotope peak clusters of a first pair of mutually associated theoretical isotope peak clusters and a second pair of mutually associated theoretical isotope peak clusters corresponds to a mass difference between two primary fragments; and

a second mass difference between non-respective mutually associated theoretical isotope peak clusters of the first pair of mutually associated theoretical isotope peak clusters and the second pair of mutually associated theoretical isotope peak clusters corresponds to the mass difference between two primary fragments.

This corresponds with the second condition.

In one example, the method comprises filtering the mutually associated theoretical isotope peak clusters to retain only the mutually associated theoretical isotope peak clusters wherein:

the respective masses of a first pair of mutually associated theoretical isotope peak clusters and of a second pair of mutually associated theoretical isotope peak clusters correspond to the same mass.

This corresponds with the third condition.

The fifteenth aspect provides a method implemented by a computer including a processor and a memory, the method comprising:

analyzing a mass spectrum according to any one or more of the first aspect to the tenth aspect;

interpreting the mass spectrum according to any one or more of the eleventh aspect to the fourteenth aspect;

proposing a candidate molecule or molecular species, for example a protein, by database searching the results of the interpreting, such as a peptide, resulting from the mutually cross-associated theoretical isotope clusters; and

optionally, confirming the candidate molecule or molecular species, by matching, for example fragment matching, using the mass spectrum.

The sixteenth aspect provides: a computer including a processor and a memory configured to perform a method according to any one or more of the first aspect to the fifteenth aspect; a computer program comprising instructions which, when executed by a computer including a processor and a memory, cause the computer to perform a method according to any one or more of the first aspect to the fifteenth aspect; and/or a non-transient computer-readable storage medium comprising instructions which, when executed by a computer including a processor and a memory, cause the computer to perform a method according to any one or more of the first aspect to the fifteenth aspect.

The seventeenth aspect provides a mass spectrometer comprising a computer including a processor and a memory configured to perform a method according to any one or more of the first aspect to the fifteenth aspect.

**BRIEF DESCRIPTION OF THE DRAWINGS**

FIG. 1 schematically depicts a method according to an exemplary embodiment;

FIG. 2 schematically depicts a method according to an exemplary embodiment;

FIG. 3 schematically depicts a method according to an exemplary embodiment;

FIG. 4 schematically depicts a method according to an exemplary embodiment;

FIG. 5 schematically depicts a method according to an exemplary embodiment;

FIG. 6 schematically depicts a method according to an exemplary embodiment;

FIG. 7 schematically depicts a method according to an exemplary embodiment;

FIG. 8 schematically depicts a method according to an exemplary embodiment; and

FIG. 9 schematically depicts a method according to an exemplary embodiment.

**DETAILED DESCRIPTION OF THE DRAWINGS**

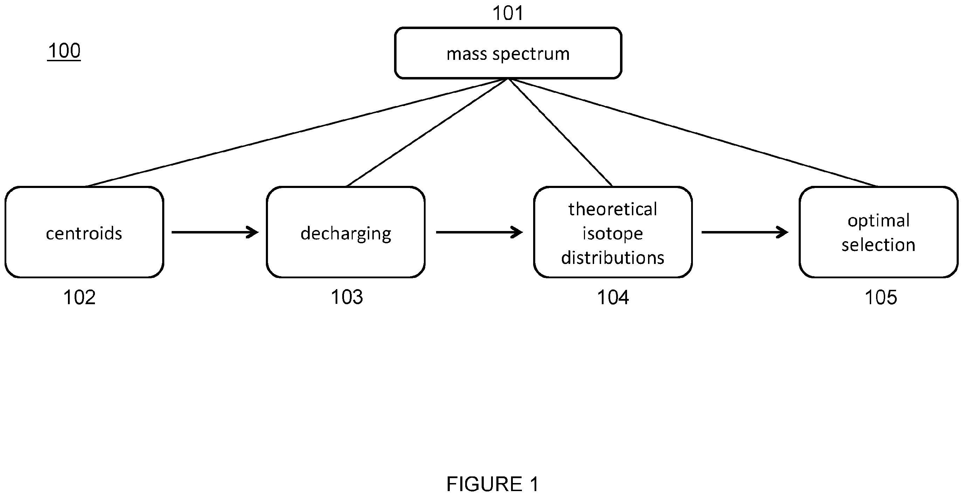

The invention comprises new algorithms for the analysis of complex mass spectra produced experimentally where spectral congestion is markedly high. Furthermore, the invention comprises new methods for processing top-down mass spectra of biopolymers, for example, DNA, RNA, proteins, glycans and oligosaccharides. Figure 1 shows a first workflow 100 of the present invention for analyzing a mass spectrum, including the steps of providing a mass spectrum 101, defining all centroids corresponding to precursor and fragment ion peaks 102, identifying all possible charge states of all the centroids present in the mass spectrum 103, calculating and applying theoretical charge state distributions to the mass spectrum 104, and ultimately identifying the optimal subset 105 of the theoretical isotope distributions that provide the best match to the fragment ion peaks and the corresponding isotope clusters.

The process of analyzing a mass spectrum 101 first comprises identifying all the centroid peaks 102 in a mass spectrum. All the following calculation steps can be applied to both centroided or profile data. In the case of analyzing profile data, a centroid (m/z value) is determined for every fragment ion peak in the mass spectrum. The mass spectrum may also contain precursor fragment ion peaks where the fragments originate from.

A subsequent processing step 103 in the algorithm is to measure all possible distances between any given centroid and the neighboring centroids that fall within an m/z range of $\pm 1.00784$, including a mass tolerance error. Quantized distances of the form $\pm 1.00784/n$, n=1, 2... k, where k is the highest charge state expected to be observed in a mass spectrum, allow calculating all the possible charge states that any given fragment ion peak can participate in. In congested mass spectra with a high degree of overlapping, it is common that a given centroid peak encompasses more than one fragment ion peaks corresponding to different isotope clusters at different charge states.

A subsequent processing step 104 in the algorithm comprises calculating a theoretical isotope distribution for every centroid peak and in all the possible charge states identified in the previous processing step 103, and further placing these theoretical isotope distributions in the mass spectrum as a first step in deciphering the experimental data. Preferably, the theoretical isotope distributions are shifted in the m/z scale to align with the centroids of the fragment ion peaks. The same calculation step can be performed on the neutral mass scale after performing a charge deconvolution step.

Theoretical isotope distributions are calculated based on chemical formulas. Each chemical formula exhibits a theoretical monoisotopic mass approximating the mass of the fragment ion peak and the corresponding isotope cluster. For example, the Averagine chemical formula can be used for analyzing protein mass spectra to calculate theoretical isotope distributions. The calculation step may also involve adjusting or optimizing the relative number of the elements present in the chemical formula to improve the goodness of fit or the alignment. Similar approaches can be adopted for different classes of molecules.

This part of the processing workflow provides one or more theoretical isotope distributions at different charge states for every fragment ion peak present in the mass spectrum, with the intensities adjusted according to the intensities of

EP 4 654 246 A1

the experimental data. All calculations are performed with a predetermined or user-adjustable mass tolerance. The calculations can be performed in the neutral mass scale and/or in the m/z scale.

A subsequent processing step 105 in the algorithm comprises providing a scoring system for ranking the theoretical isotope distributions based on several features of the mass spectrum, including but not limited to mass accuracy, peak alignment, peak intensity, isotope ratios, area-under-the-curve, correlation etc.

Examples of the different scores that can be implemented in the algorithm are provided here:

Mass accuracy or mass tolerance - based scores

$e_i$ = Percentage of total centroid intensity of theoretical isotope distribution falling within ppm mass accuracy threshold. Provides a qualitative assessment of each theoretical isotope distribution based on mass accuracy error.

$$e_i = 1 - \frac{\sum_p^{missing\ peaks} I_p}{\sum_{p_t}^{total\ peaks} I_{p_t}}$$

$I_p, I_{pt}$ : intensities of peaks $p$ and $p_t$

Al = intensity weighted goodness of alignment of each theoretical centroid to its corresponding experimental. Qualitative assessment of each theoretical isotope distribution based on mass accuracy error. In this function the score gives a measure of the collective position of the centroids relative to the experimental centroids.

$$Al = \sum_p w_p \cdot erfc(\frac{m_p - m_{p_{al}}}{\sqrt{2}\sigma_p})$$

$w_p$ : intensity-based weight of the peak ρ

$m_p$ : m/z of the peak $p$ and its aligned $p_{al}$

Intensity - based scores

$m_i$ = Intensity coverage only in the areas under each centroid "bell" curve

$$m_i = 1 - \frac{\sum_{i=0}^{N} min(y_{t_i}, y_{e_i})}{\sum_{i=0}^{N} y_{e_i}} \in [0,1]$$

N : number of aligned points

$y_e, y_t$ : vectors of experimental and theoretical intensities

Cov = Intensity coverage under the whole span of the theoretical isotope distribution

$$Cov = \frac{\sum_{i=0}^{K} min(y_{t_i}, y_{e_i})}{\sum_{i=0}^{N} y_i}$$

K : number of all data points within the theoretical distribution m/z range

$y_e, y_t$ : vectors of experimental and theoretical intensities

Ratio = intensity weighted assessment of the ratios of all successive theoretical centroids within mass accuracy based on the experimental centroid ratios

$$Ratio = \prod_i^{\#aligned\ peaks} w_p \cdot erfc(\frac{R_t(i, i-1) - R_e(i, i-1)}{\sqrt{2}\sigma_r})$$

14

$$R(x,y): \frac{I_x}{I_y}$$

$w_p$ : weight of the ratio, based on the sum of two peaks $\quad$ between two peaks of a distribution

$\sigma_r$ : standard deviation of $R(x,y)$ ratios between two distributions (calculated using Taylor expansion)

local int = the maximum intensity the theoretical distribution can reach, given the experimental local maximum intensity.

$$local\ int = \frac{I_{th\_max}}{I_{exp\_max}}$$

$I_{th\_max}$: maximum intensity that the theoretical isotope distribution can reach when its amplitude is automatically adjusted to the experimental data

$I_{exp\_max}$: maximum experimental intensity wherever $I_{th} > 0$

**global int** = Given an intensity value **int,** the global intensity score estimates the probability of observing an intensity larger than **int** in the spectrum. Estimate the CDF (Cumulative Distribution Function) $F_I(i)$ of the intensities of the mass spectrum. The score is then:

$$global\ int = 1 - F_I(int) = 1 - P(I \le int)$$

where:

- $I$ = the random variable representing the intensity of the spectrum
- $P(I \le int)$ = the probability that I will take on a value less than **int**

**Correlation** = a statistical measure of the strength of association between two isotope distributions. The correlation coefficient expresses the linear correlation between the theoretical isotope distribution and corresponding experimental intensity.

$$Correlation = \frac{\sigma_{et}}{\sigma_e \sigma_t} \in [-1,1]$$

$$\mu_e = \frac{\sum_i^N w_i I_{e,i}}{\sum_i^N w_i}, \mu_t = \frac{\sum_i^N w_i I_{t,i}}{\sum_i^N w_i}$$

$$\sigma_e = \sqrt{\frac{\sum_i^N w_i (I_{e,i} - \mu_e)^2}{\sum_i^N w_i}}, \sigma_t = \sqrt{\frac{\sum_i^N w_i (I_{t,i} - \mu_t)^2}{\sum_i^N w_i}}$$

$$\sigma_{et} = \sqrt{\frac{\sum_i^N w_i (I_{e,i} - \mu_e)(I_{t,i} - \mu_t)}{\sum_i^N w_i}}$$

$I_{e,i}$: experimental intensity on index i

$I_{t,i}$: theoretical intensity on index i

The scoring system is used for ranking the theoretical isotope distributions, for example, either based on a mass and/or a mass-to-charge ratio difference between a theoretical isotope and a corresponding fragment ion peak, and/or based on the difference in the relative abundance between at least two neighboring theoretical isotopes and the corresponding fragment ion peaks, and/or based on differences in intensity and/or differences in the area-under-the-curve.

A single theoretical isotope distribution with the highest score is selected and fitted in the mass spectrum for every individual fragment ion peak to form a first selected subset of theoretical isotope distributions, and with each theoretical isotope distribution potentially assigned to more than one fragment ion peaks. The next calculation step involves subtracting the area under the first subset of the selected theoretical isotope distributions with the highest score from the fragment ion peaks, to produce a residual profile mass spectrum including partially assigned peaks with reduced intensity.The remaining theoretical isotope distributions that were not selected in the first assignment or fitting step are provided in the next iteration and the assignment or fitting process is repeated until a set of convergence criteria is satisfied.

The fitting process is completed when at least one convergence criterion is satisfied, for example, when there are no remaining fragment ion peaks in the residual mass spectrum above a predetermined noise threshold. Different or additional convergence criteria can be implemented, for example when the change in the intensity of the theoretical isotope distributions in two successive iterations falls below a predetermined value. A preferred convergence criterion is having the relative change in the difference between the intensities of the theoretical isotope distributions and the intensities of the fragment ion peaks in two consecutive iteration steps falling below a predetermined threshold.

The analysis according to a first aspect of the present invention provides a list or a compilation of theoretical isotope distributions fitted in the mass spectrum that provide the most accurate representation of the fragment ions peaks and corresponding isotope clusters. The theoretical isotope distributions disentangle the congested regions of overlapping isotope clusters with high precision, also providing an accurate measurement of the monoisotopic mass of all the isotope clusters corresponding to fragments.

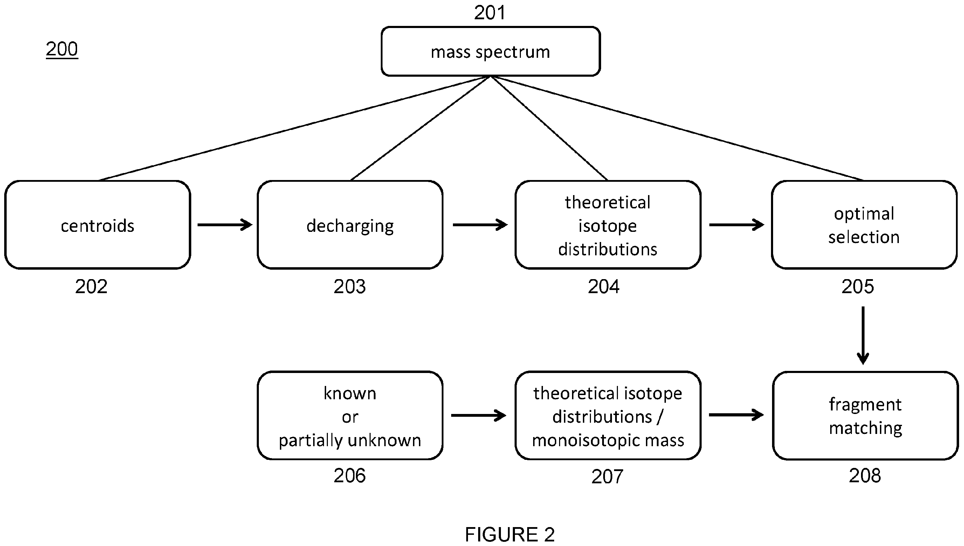

The workflow presented in Figure 1 can be applied for analyzing a mass spectrum of an unknown molecule or precursor ions. The same workflow can be applied for analyzing a mass spectrum of a known or partially unknown molecule, for example, a protein with a known or partially unknown amino acid sequence, with a few additional steps as shown in Figure 2.

A second workflow 200 of the present invention for analyzing a mass spectrum of a known or partially unknown molecule, includes the steps of providing a mass spectrum 201, defining all centroids corresponding to precursor and fragment ion peaks 202, identifying all possible charge states of all the centroids present in the mass spectrum 203, calculating and applying theoretical isotope distributions to the mass spectrum 204, for example using the Averagine chemical formula in the case of proteins, and ultimately identifying the optimal subset 205 of the theoretical isotope distributions that provide the best match to the fragment ion peaks and the corresponding isotope clusters.

The steps described so far with reference to Figure 2 are equivalent to the steps presented in Figure 1. Additional calculations steps are provided if the identity of the molecule is partially unknown 206, for example, a known protein sequence with one or more unknown modifications. In such case, the method relies in calculating all the possible theoretical fragments 207 for every protein variant and further determines which set of theoretical fragments corresponding to a single variant is the best match 208 to the optimal selection 205. The same workflow can be applied to a mass spectrum originating from a known molecule, for example, a protein with a known amino acid sequence.

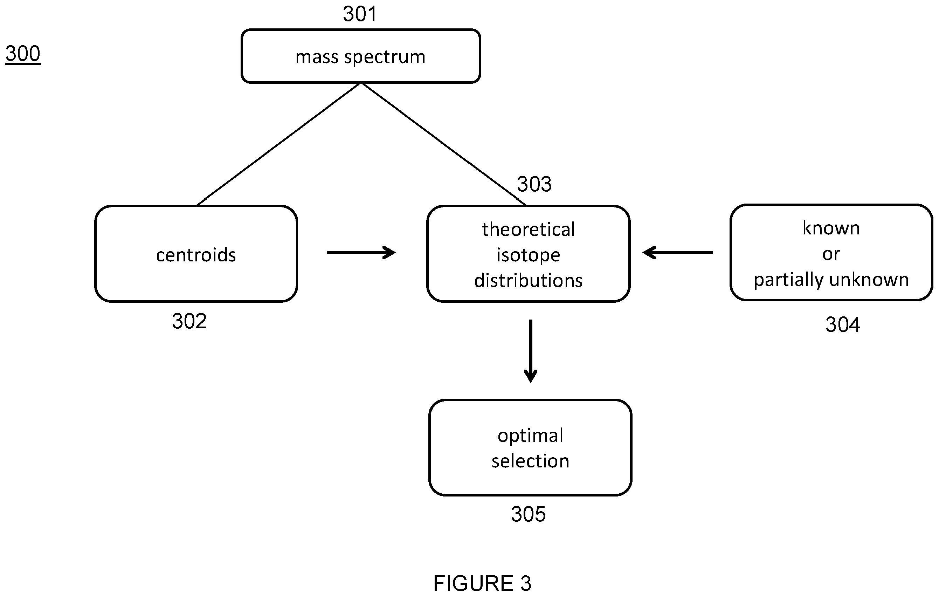

Figure 3 shows a third workflow 300 of the present invention for analyzing a mass spectrum of a known or partially unknown molecule, and includes the steps of providing a mass spectrum 301, defining all centroids corresponding to precursor and fragment ion peaks 302, calculating and applying theoretical charge state distributions for a known or partially unknown molecule 304, for example, using the chemical formulas of the theoretical fragments based on the amino acid sequence of a known or a partially unknown protein, and ultimately identifying the optimal subset 305 of the theoretical isotope distributions that provide the best match to the fragment ion peaks and the corresponding isotope clusters.

The present invention also provides a new method for analyzing a mass spectrum *de novo.* The method comprises creating a graph consisting of a set of nodes connected through a set of edges, with each node representing the (monoisotopic) mass of a theoretical isotope distribution, and with the edges of the graph representing the difference in the (monoisotopic) mass between two nodes.

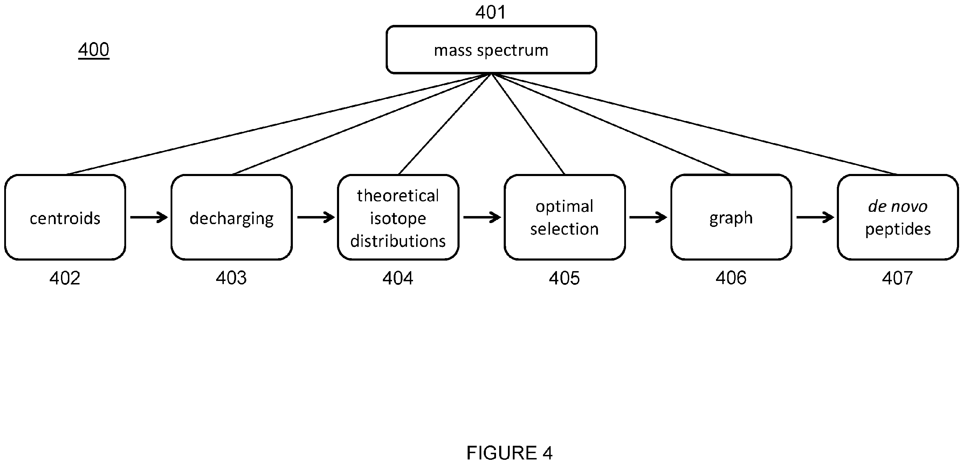

Figure 4 is an example of a workflow 400 for generating a list of *de novo* peptides 407 from a fragment ion mass spectrum 401, according to the eleventh, twelfth and/or thirteenth aspect. Steps 402 - 405 are equivalent to steps 102 - 105 described with reference to Figure 1. The next step in the *de novo* sequencing workflow 400 is mutually associating the selected theoretical isotope peak clusters, for example by generating a graph 406 based on the monoisotopic masses of the optimal selection of the theoretical isotope distributions that provide the most accurate representation 405 of the fragment ion mass spectrum 401. The algorithm calculates and stores the mass difference between any two nodes in the graph matching a predetermined list of masses. For example, if an intact protein is fragmented producing a top-down mass spectrum, the predetermined list of masses includes the masses of the standard twenty amino acids. The list can be modified and/or extended to include amino acids with modified side chains to identify post translational modifications, for example.

In this example, a list of *de novo* peptides 407 is generated with the first amino acid in every peptide starting from a root node representing a node with the smallest (monoisotopic) mass. There can be one or more node paths originating from the root note, and with the edges connecting the nodes defining the one or more node paths, while each path corresponds to a different *de novo* peptide.

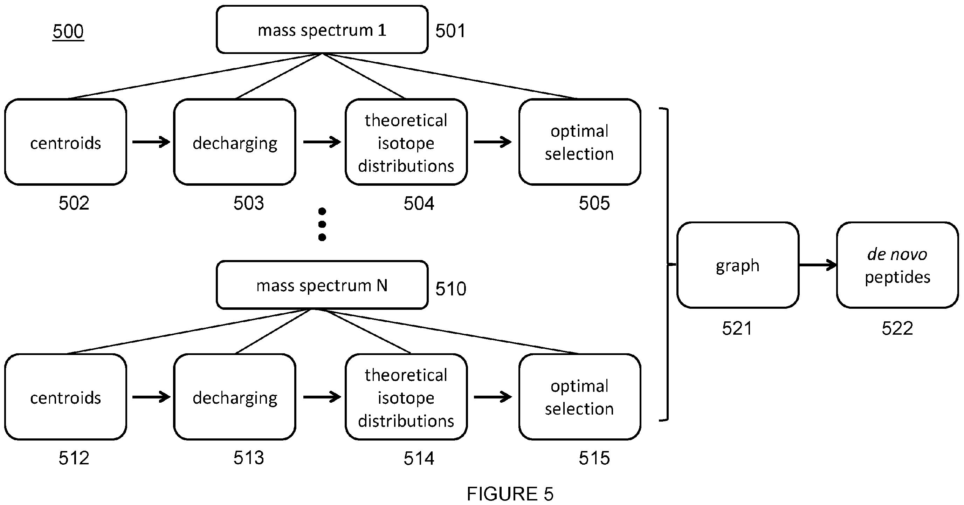

The workflow 500 presented in Figure 5 is an extension of the workflow 400, with steps 502 - 505 and steps 512 - 515 being equivalent to steps 402 - 405 and corresponding to a first mass spectrum 501 and to an Nth mass spectrum 520 respectively, where N≥2. If the first to the Nth mass spectrum corresponds to the same analyte or the same precursor ion, the optimal selection of the theoretical isotope distributions from the first 505 to the Nth spectrum 515 can be combined to generate a single graph 521 (i.e. mutually cross-associating the mutually associated theoretical isotope peak clusters) and produce the combined list of *de novo* peptides 522. The purpose of combining information from multiple spectra for the same molecular identity or the same analyte is to increase the density and the quality of information that can be further processed or exploited to eliminate false positive *de novo* peptides from the output list 522 of the algorithm.

In a first example, different charge states of the same protein precursor can be isolated in a mass spectrometer and subsequently subjected to the same dissociation step to generate different fragment ion mass spectra, where most of the fragments in each mass spectrum are observed at different charges states. This relationship between same mass fragments observed at different charges states in different spectra can be translated into a graph, as shown in Figure 5, and further exploited to improve the confidence in the results of the *de novo* workflow, as it will become apparent from the detailed description of the new filtering methods disclosed below.

In another example, different fragmentation methods are applied to the same charge state or to different charge states of the same protein precursor to generate different fragment ion mass spectra, where most of the fragments in each mass spectrum are observed in equivalent charge states but in different fragment types or forms, for example, in most or all the six primary fragment forms known in tandem MS, namely, a, b, c and x, y, z. The relationship between fragments ions of the same mass observed in different spectra at equivalent charges state but in different forms can be translated into a graph, as shown in Figure 5, and further exploited to improve the confidence in the results of the *de novo* workflow, as it will become apparent from the detailed description of the new filtering methods disclosed below.

In yet another example, MS2 and MS3 data from the same precursor can be combined to expand the graph and improve the confidence in the results of the *de novo* workflow. For example, MS3 analysis of selected MS2 fragments of a protein can be performed and the data can be combined to enhance the coverage in specific regions of the amino acid sequence. Different combinations of fragment ion mass spectra can be considered to improve the confidence in the results of the *de novo* workflow, without departing from the scope of the present invention.

The present invention further provides a combination of new methods for reducing the number of false positive *de novo* peptides produced in the workflows presented in Figures 4 and 5.

Complementarity or bidirectional rule (first condition)

The list of *de novo* peptides produced in steps 407 and 522 can be filtered by utilizing complementary fragment ion information. Since the mass of the precursor ion is known within a narrow mass tolerance range, pairs of fragments can be identified such that their combined mass matches the mass of the precursor. For example, N- and C-terminal fragment pairs in a protein have a total mass that corresponds to the mass of the protein. Figure 6 shows a narrow segment of a graph 600 with two different node paths originating from root nodes 601 and 611 respectively. In this example, the mass of the theoretical isotope distribution represented by node 602 and the mass of the theoretical isotope distribution represented by node 613 add up to the mass of the precursor ion M. The confidence in assigning the amino acid AAi indicated by the edges connecting nodes 602 - 603 and 612 -613 is considerably enhanced if the mass of the theoretical isotope distribution represented by node 603 and the mass of the theoretical isotope distribution represented by node 612 also add up to the mass of the precursor ion M. The list of *de novo* peptides can therefore be filtered by selecting only those amino acids that satisfy such a condition, that is, there exists two pairs of nodes, 602, 603 and 612, 613 having the same mass difference corresponding to the mass of an amino acid, AAi, and the pair of nodes further satisfy the condition that a first node 602 from the first pair and a first node 613 from the second pair have a total mass corresponding to the mass of the precursor ion M, while the second node 603 from the first pair and the second node from the second pair 612 also have a total mass corresponding to the mass of the precursor ion M. In this example, the node path originating from the root node 601 could correspond to N-terminal b-type fragments, while the node path originating from the root node 611 would correspond to C-terminal y-type fragments.

Unidirectional or fragment delta rule (second condition)

The list of *de novo* peptides produced in steps 407 and 522 can be filtered by utilizing additional fragment ion information related to constant differences in mass and/or constant mass shifts. Figure 7 shows a narrow segment of a graph 700 with two different node paths originating from root nodes 701 and 711 respectively. In this example, nodes 702 and 703 and nodes 712 and 713 have the same mass difference corresponding to the mass of an amino acid AAi. Simultaneously, the mass difference between nodes 702 and 712 is equivalent to the mass difference between nodes 703 and 713. This mass difference can be matched to the mass difference between two different fragment types, for example, the mass difference between b-type and a-type fragments. This method can be extended to any mass difference between N-terminal fragments (a, b, c) and to any mass difference between C-terminal fragments (x, y, z) to filter the list of *de novo* peptides. The same method can be applied to other less commonly reported fragment types (d, v, w) as well as by utilizing constant differences in mass between primary and internal fragment ladders. The confidence in assigning the amino acid AAi indicated by the edges connecting nodes 702 - 703 and 712 - 713 is considerably enhanced if such a condition is satisfied, namely, there exists a pair of nodes 702, 703 and 712, 713 having the same mass difference corresponding to the mass of an amino acid, AAi, and the pair of nodes further satisfy the condition that the mass difference between a first node 702 in the first pair of nodes and a first node 712 in a second pair of nodes equals the mass difference between the second node 703 in the first pair of nodes and the second node 713 in a second pair of nodes.

The constant difference in mass between the different fragment types can be exploited to identify the fragment type in a fragment ladder comprising a series of sequential fragments of the same type or form corresponding to a region of the amino acid sequence. This information can distinguish between N-terminal and C-terminal fragments and further define the direction of a peptide in a protein, for example. The direction is essential for aligning *de novo* peptides and building up the amino acid sequence of a protein.

The *de novo* algorithm disclosed in the present invention also allows for transitioning between a first fragment type and a second fragment type in defining a node path and ultimately reporting a peptide. Figure 7 is an example where the node path originating from root node 701 comprises a fragment ladder of b-type ions, while the node path originating from root node 711 comprises a fragment ladder of a-type ions. Transitions between the different node paths can be made if the list of masses, in this example, the list of the known amino acids and modifications thereof, also include amino acids with mass differences corresponding to the mass differences between fragment types. For example, a peptide may correspond to a ladder of b-type fragment ions with a fraction of these fragments satisfying the b-a condition, as shown in Figure 7, while the remaining fraction will satisfy the c-b mass difference. This method can be used to increase the number of connections between the nodes in a graph to produce additional and possibly longer peptides. These additional peptides where transitions between different fragment types are allowed can also be combined with different rules, for example the complementarity rule described above, and the multi-z rule disclosed further below.

Additional relationships between fragment types can be used to identify amino acids with high confidence. For example, the formation of a d-type fragment can be correlated to an N-terminal primary fragment (a, b, c), which will provide an additional measure for confirming the identity of a given amino acid. Similarly, a w-type and v-type fragment can be correlated to a C-terminal primary fragment (x, y, z). The mass differences between primary fragments and fragments with side chain losses such as the d, v and w ions can be combined further with the filtering aspects disclosed in the present invention to identify amino acid sequences with high confidence.

Multi-z or charge-state rule (third condition)

The list of *de novo* peptides produced in steps 407 and 522 can be filtered by utilizing additional fragment ion information related to equal mass fragments observed at different charge states. Figure 8 shows a narrow segment of a graph 800 with two different node paths originating from root nodes 801 and 811 respectively. In this example, the first node path corresponds to theoretical isotope distributions at charge state n+ and the second node path corresponds to theoretical isotope distributions at charge state (n+1)+, while the edges connecting the node pairs 802 - 803 and 812 - 813, with each pair located in a separate node path at a different charge state, are equal and correspond to the mass of an amino acid AAi. Simultaneously, a first node 802 of the first pair of nodes and a first node 812 of the second pair of nodes have a first equivalent neutral mass, while the second node of the first pair of nodes and the second node of the second pair of nodes have a second equivalent neutral mass. This condition is satisfied if the first nodes in both pair of nodes correspond to fragment ions observed at different charge states but have a first equivalent neutral mass, and the second nodes in both pair of nodes correspond to fragment ions observed at different charge states but have a second equivalent neutral mass.

The present invention further provides an additional method for reducing the number of false positive *de novo* peptides listed by combining any two of the three filtering aspects disclosed with reference to Figures 6, 7 and 8. The method further includes combining all three filtering aspects to produce a list of *de novo* peptides.

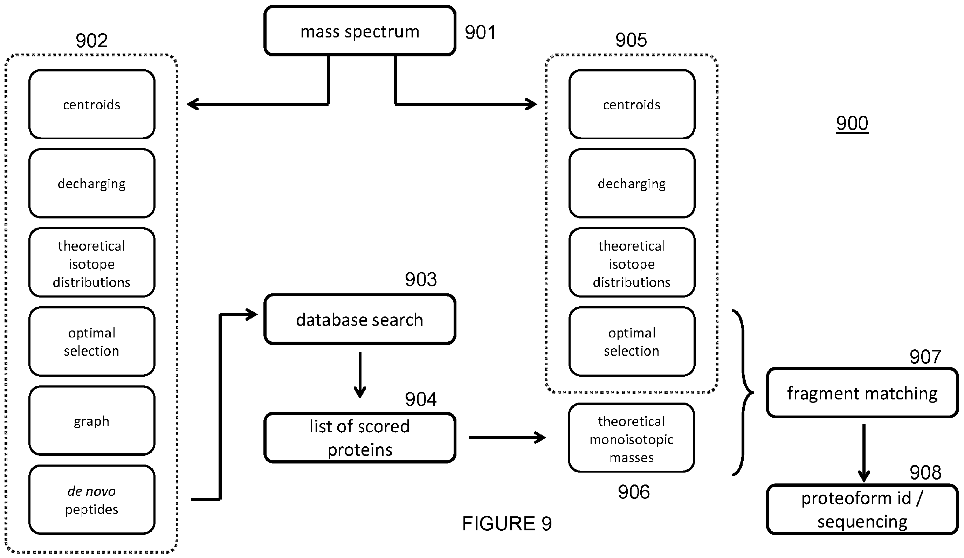

Figure 9 shows yet another workflow 900 of the present invention, particularly a method according to the fifteenth aspect, which incorporates the workflows disclosed with reference to Figures 2 and 4 and applied for the analysis and identification of unknown molecules or analytes. The first part of the workflow 902 includes the steps of providing a mass spectrum 901, defining all centroids corresponding to precursor and fragment ion peaks, identifying all possible charge states of all the centroids present in the mass spectrum, calculating and applying theoretical isotope distributions to the mass spectrum, for example using the Averagine chemical formula in the case of proteins, and ultimately identifying the optimal subset of the theoretical isotope distributions that provide the most accurate representation of the fragment ion peaks and the corresponding isotope clusters. The next step of this part of the workflow 902 is generating a graph based on the monoisotopic masses of the optimal selection of the theoretical isotope distributions, and subsequently producing a list of *de novo* peptides that is preferably filtered based on one or more conditions disclosed with reference to Figures 6, 7 and 8.

The next steps in the workflow presented in Figure 9 involve submitting the list of *de novo* peptides to a protein database 903 and acquiring a list of relevant proteins provided with a matching score 904.

The second part of the workflow 905 includes equivalent steps to those performed in the *de novo* part of the workflow 902 for centroiding, decharging, providing theoretical isotope distributions based on the Averagine chemical formula and calculating the optimal subset providing the best fit.

The final step in this exemplary workflow is to calculate the monoisotopic masses 906 for every relevant protein 904 provided by the database search 903, and subsequently to perform a fragment matching calculation step where the monoisotopic peaks from the optimal subset or optimal selection 905 is matched to the list of monoisotopic masses 906. The fragment matching approach will confirm the protein that provides the best match, which may differ from the highest scoring protein provided by the database search using a list of *de novo* peptides. Furthermore, the spectral matching step of this workflow can be further expanded to include additional modifications to the proteins provided by the database search and to possibly identify the protein variant, as escribed with reference to Figure 2. The workflow 900 can be combined with other workflows disclosed in the present invention, for example the workflow described with reference to Figure 5 where MS2 data from different types of fragmentation mechanisms or from different charge states of the same analyte/molecule are combined to enhance the result of the *de novo* peptide list.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

**Claims**

1. A method of analyzing mass spectra, the method implemented by a computer including a processor and a memory, the method comprising steps:

    (i) obtaining a mass spectrum comprising a series of isotope peak clusters, including a first isotope peak cluster and a second isotope peak cluster, corresponding to respective product ions of a precursor ion;
    (ii) calculating theoretical isotope peak clusters for the series of isotope peak clusters;
    (iii) fitting the theoretical isotope peak clusters to the series of isotope peak clusters;
    (iv) ranking the theoretical isotope peak clusters, based on respective results of the fitting;
    (v) selecting the theoretical isotope peak clusters, based on respective results of the ranking; and
    (vi) generating a residual mass spectrum from the mass spectrum, using the selected theoretical isotope peak clusters;
(vii) repeating steps (iii) to (vi) for the residual mass spectrum; and
(viii) providing/outputting/storing the selected theoretical isotope peak clusters.

2. The method according to claim 1, comprising determining the respective monoisotopic masses of first isotope peak cluster and the second isotope peak cluster.

3. The method according to any previous claim, comprising centroiding the mass spectrum and/or decharging the mass spectrum.

4. The method according to any previous claim, wherein calculating the theoretical isotope peak clusters for the series of isotope peak clusters comprises calculating the theoretical isotope peak clusters for a plurality of peaks, optionally for each peak, of the series of isotope peak clusters.

5. The method according to any previous claim, wherein calculating the theoretical isotope peak clusters for the series of isotope peak clusters comprises predicting respective chemical formulae for the series of isotope peak clusters, for example for a plurality of peaks, optionally for each peak, of the series of isotope peak clusters.

6. The method according to any previous claim, wherein fitting the theoretical isotope peak clusters to the series of isotope peak clusters comprises shifting, optionally aligning, the theoretical isotope peak clusters relative to the series of isotope peak clusters (i.e. m/z).

7. The method according to any previous claim, wherein fitting the theoretical isotope peak clusters to the series of isotope peak clusters comprises scaling (i.e. intensities) the theoretical isotope peak clusters relative to the series of isotope peak clusters.

8. The method according to any previous claim, wherein fitting the theoretical isotope peak clusters to the series of isotope peak clusters comprises iteratively and/or simultaneously fitting the theoretical isotope peak clusters to the series of isotope peak clusters.

9. The method according to any previous claim, wherein generating the residual mass spectrum from the mass spectrum, using the theoretical isotope peak clusters, comprises determining a residual value for the residual mass spectrum.

10. The method according to any previous claim, wherein repeating steps (iii) to (vi) for the residual mass spectrum comprises repeating steps (iii) to (vi) for the residual mass spectrum while the residual value is greater than a predetermined threshold.

11. A method of interpreting mass spectra, the method implemented by a computer including a processor and a memory, the method comprising steps:

(1) obtaining theoretical isotope peak clusters for a mass spectrum, for example by analyzing the mass spectrum according to any of claims 1 to 10;
(2) mutually associating the theoretical isotope peak clusters, based on a set of predetermined masses, for example the masses of the twenty amino acids and/or modifications thereof;
(3) mutually cross-associating the mutually associated theoretical isotope peak clusters, based on precursor ion information and/or product ion information, for example product ion patterns; and
(4) interpreting the mass spectrum, based on the mutually cross-associated theoretical isotope peak clusters.

12. The method according to claim 11, comprising filtering the mutually associated theoretical isotope peak clusters to retain only the mutually associated theoretical isotope peak clusters wherein:

the total mass of a first pair of mutually associated theoretical isotope peak clusters corresponds to a known precursor mass;
the total mass of a second pair of mutually associated theoretical isotope peak clusters corresponds to the known precursor mass; and
a mass difference between the respective mutually associated theoretical isotope peak clusters of the first pair of mutually associated theoretical isotope peak clusters and the second pair of mutually associated theoretical isotope peak clusters corresponds to the same mass in the set of predetermined masses.

13. The method according to any of claims 11 to 12, comprising filtering the mutually associated theoretical isotope peak clusters to retain only the mutually associated theoretical isotope peak clusters wherein:

a first mass difference between non-respective mutually associated theoretical isotope peak clusters of a first pair of mutually associated theoretical isotope peak clusters and a second pair of mutually associated theoretical isotope peak clusters corresponds to a mass difference between two primary fragments; and
a second mass difference between non-respective mutually associated theoretical isotope peak clusters of the first pair of mutually associated theoretical isotope peak clusters and the second pair of mutually associated theoretical isotope peak clusters corresponds to the mass difference between two primary fragments.

14. The method according to any of claims 11 to 13, comprising filtering the mutually associated theoretical isotope peak clusters to retain only the mutually associated theoretical isotope peak clusters wherein:
the respective masses of a first pair of mutually associated theoretical isotope peak clusters and of a second pair of mutually associated theoretical isotope peak clusters correspond to the same mass.

15. A method implemented by a computer including a processor and a memory, the method comprising:

analyzing a mass spectrum according to any of claims 1 to 10;
interpreting the mass spectrum according to any of claims 11 to 14;
proposing a candidate molecule or molecular species, for example a protein, by database searching the results of the interpreting, such as a peptide, resulting from the mutually cross-associated theoretical isotope clusters; and
optionally, confirming the candidate molecule or molecular species, by matching, for example fragment matching, using the mass spectrum.

100

101 mass spectrum

102 centroids

103 decharging

104 theoretical isotope distributions

105 optimal selection

FIGURE 1

EP 4 654 246 A1

201

mass spectrum

200

| centroids | → | decharging | → | theoretical isotope distributions | → | optimal selection |

202 · 203 · 204 · 205

| known or partially unknown | → | theoretical isotope distributions / monoisotopic mass | → | fragment matching |

206 · 207 · 208

FIGURE 2

300

301 mass spectrum

302 centroids

303 theoretical isotope distributions

304 known or partially unknown

305 optimal selection

FIGURE 3

FIGURE 4

FIGURE 5

500

mass spectrum 1 — 501

centroids — 502

decharging — 503

theoretical isotope distributions — 504

optimal selection — 505

mass spectrum N — 510

centroids — 512

decharging — 513

theoretical isotope distributions — 514

optimal selection — 515

graph — 521

*de novo* peptides — 522

FIGURE 6

FIGURE 7

FIGURE 8

900

901 mass spectrum

905
centroids
decharging
theoretical isotope distributions
optimal selection

906 theoretical monoisotopic masses

907 fragment matching

908 proteoform id / sequencing

902
centroids
decharging
theoretical isotope distributions
optimal selection
graph
*de novo* peptides

903 database search

904 list of scored proteins

FIGURE 9

Europäisches
Patentamt

European
Patent Office

Office européen
des brevets

**EUROPEAN SEARCH REPORT**

**Application Number**

EP 24 17 8105

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MICHAEL R. HOOPMANN ET AL: "High-Speed Data Reduction, Feature Detection, and MS/MS Spectrum Quality Assessment of Shotgun Proteomics Data Sets Using High-Resolution Mass Spectrometry", ANALYTICAL CHEMISTRY, vol. 79, no. 15, 21 June 2007 (2007-06-21), pages 5620-5632, XP055214728, ISSN: 0003-2700, DOI: 10.1021/ac0700833 * the whole document * * especially Fig. 1-3, p. 5622, left col. - p. 5624, left col. and p. 5630, left col. * | 1-10 | INV. H01J49/00 G01N33/68 G16B40/10 G16C20/20 |
| | ----- | | |
| | | | TECHNICAL FIELDS SEARCHED (IPC) |
| | | | H01J G01N G16C G06F G16B |

The present search report has been drawn up for all claims

| Place of search | Date of completion of the search | Examiner |
|---|---|---|
| The Hague | 8 November 2024 | Dietsche, Rainer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**Application Number**

EP 24 17 8105

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-10

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

**Application Number**

EP 24 17 8105

The Search Division considers that the present European patent application does not comply with the requirements of unity of invention and relates to several inventions or groups of inventions, namely:

> 1. claims: 1-10
>
>> method of analyzing mass spectra resulting in theoretical isotope peak clusters for the mass spectrum
>> ---
>
> 2. claims: 11-15
>
>> method of interpreting mass spectra resulting in the identification of the molecular species
>> ---